# EUROPEAN PATENT SPECIFICATION

(11) **EP 3 315 612 B1**
(45) Date of publication and mention of the grant of the patent: **17.03.2021**
(21) Application number: 16813831.1
(22) Date of filing: 24.06.2016
(51) Int. Cl.: C12Q 1/6888

(54) **SET OF PRIMERS AND METHOD FOR DETECTING AND IDENTIFYING MUSSEL SPECIES OF THE GENUS MYTILUS**
PRIMERSET UND VERFAHREN ZUR ERKENNUNG UND IDENTIFIZIERUNG VON MUSCHELN DER GATTUNG MYTILUS
ENSEMBLE D'AMORCES ET PROCÉDÉ DE DÉTECTION ET D'IDENTIFICATION D'ESPÈCES DE MOULES DU GENRE MYTILUS

(30) Priority: 24.06.2015 CL 201501833
(43) Date of publication of application: 02.05.2018
(73) Proprietor: Universidad De Chile, Santiago 8330111 (CL)
(72) Inventor: LARRAÍN BARTH, María Angélica Luisa, Santiago 8380494 (CL); JILBERTO VALLEJOS, Felipe Ignacio, Santiago 8380494 (CL); ARANEDA TOLOSA, Cristian Manuel, Santiago 8380494 (CL)
(74) Representative: Clarke Modet & Co.
(86) International application number: PCT/IB2016/053789
(87) International publication number: WO 2016/207857

(56) References cited:
- JP-A- 2011 097 922
- KOJI INOUE ET AL: "Interspecific variations in adhesive protein sequences of Mytilus edulis, M. galloprovincialis, and M. trossulus", BIOLOGICAL BULLETIN, LANCASTER PRESS, INC., LANCASTER, PA, US, vol. 189, no. 3, 1 January 1995 (1995-01-01), pages 370-375, XP009507929, ISSN: 0006-3185, DOI: 10.2307/1542155
- Maria Angelica Larrain ET AL: "Genetic composition of Mytilus species in mussel populations from southern Chile", Latin American Journal of Aquatic Research, vol. 40, n. 4, 1 January 2012 (2012-01-01), pages 1077-1084, XP055505071, DOI: 10.3856/vol40-issue4-fulltext-23 Retrieved from the Internet: URL:https://scielo.conicyt.cl/pdf/lajar/v4 0n4/art23.pdf [retrieved on 2018-09-06]
- Vassilenko E.: "Developing Biomarkers for Mussel Leukemia as Tools for Ecosystem Health Monitoring", Doctoral Thesis , 1 June 2012 (2012-06-01), XP55504985, Retrieved from the Internet: URL:https://open.library.ubc.ca/media/down load/pdf/24/1.0059270/1 [retrieved on 2018-09-06]
- Dwiyitno: "Application of real-time PCR for fish and seafood authentication", the 4th Indonesian biotechnology conference, 7 August 2008 (2008-08-07), pages 267-276, XP055520922, Retrieved from the Internet: URL:https://www.researchgate.net/publicati on/303381178_Application_of_Real_Time_PCR_ for_Fish_and_Seafood_Authentication [retrieved on 2018-11-05]
- INOUE, K . ET AL.: 'Interspecific variations in adhesive protein sequences of Mytilus edulis, M. galloprovincialis, and M. trossulus.' THE BIOLOGICAL BULLETIN vol. 189, no. 3, 1995, pages 370 - 375, XP009507929
- VASSILENKO, E.: 'Developing biomarkers for mussel leukemia as tools for ecosystem health monitoring.' TESIS DOCTORAL June 2012, XP055504985 Retrieved from the Internet: <URL:https://open.library.ubc.ca/media/down load/pdf/24/1.0059270/1> [retrieved on 2016-09-21]
- MAO, J. ET AL.: 'Development of a Rapid and Efficient Method for Non-Lethal DNA Sampling and Genotyping in Scallops.' PLOS ONE vol. 8, no. 7, 2013, page E68096, XP055505004
- DIAS, P. J. ET AL.: 'Development of a real-time PCR assay for detection of Mytilus species specific alleles: application to a sampling survey in Scotland.' JOURNAL OF EXPERIMENTAL MARINE BIOLOGY AND ECOLOGY vol. 367, no. 2, 2008, pages 253 - 258, XP025679994
- LARRAIN, M. A. ET AL.: 'Genetic composition of Mytilus species in mussel populations from southern Chile.' LATIN AMERICAN JOURNAL OF AQUATIC RESEARCH vol. 40, no. 4, 2012, pages 1077 - 1084, XP055505071
- GONZALEZ, P.: 'Identificación de especies del género Mytilus utilizando marcadores moleculares mitocondriales y nucleares.' TESIS MAESTRIA XP055505080 Retrieved from the Internet: <URL:http://repositorio.uchile.cl/handle/22 50/138249> [retrieved on 2016-09-21]
- FERNANDEZ-TAJES, J. ET AL.: 'Alternative PCR-RFLP methods for mussel Mytilus species identification.' EUROPEAN FOOD RESEARCH AND TECHNOLOGY vol. 233, no. 5, 2011, pages 791 - 796, XP019970005
- ZBAWICKA, M. ET AL.: 'Identification and validation of novel SNP markers in European populations of marine Mytilus mussels.' MARINE BIOLOGY vol. 159, no. 6, 2012, pages 1347 - 1362, XP035048750

### SEQUENCE LIST

<110> UNIVERSITY OF CHILE
<120> SET OF PRIMERS AND METHOD FOR DETECTING
   AND IDENTIFYING MUSSEL SPECIES OF THE GENUS
   MYTILUS, USING HIGH-RESOLUTION MELTING AND PCR
<130> W 2016/7563
<150> CL 2015-01833
   <151> 24-06-2015
<160> 5
<170> Patentln version 3.5
<210> 1
   <211> 18
   <212> DNA
   <213> Mytilus
<400> 1
   ggaacaaagc atggacca 18
<210> 2
   <211> 20
   <212> DNA
   <213> Mytilus
<400> 2
   gacagcttct ttgcaagtgg 20
<210> 3
   <211> 116
   <212> DNA
   <213> Mytilus galloprovincialis
<400> 3
   ggaacaaagc atggaccagt atacaaacct gtgaagacaa gttatcatcc tacgaatagt 60
   tatccgccaa catatggatc aaagacaaac tatctgccac ttgcaaagaa gctgtc 116
<210> 4
   <211> 116
   <212> DNA
   <213> Mytilus chilensis
<400> 4
   ggaacaaagc atggaccagt atacaaacct gtgaagacaa gttatcatcc tacgaatagt 60
   tatcctccaa catatggatc aaagacaaac tatctgccac ttgcaaagaa gctgtc 116
<210> 5
   <211>170
   <212> DNA
   <213> Mytilus edulis
<400> 5

## Description

### FIELD OF THE INVENTION

The present invention refers to a set of specific primers and to the use thereof in polymerase chain reaction (PCR) together with the "High Resolution Melting" (HRM) technique for identifying mussel species of the genus *Mytilus* in a rapid and less costly manner. The method disclosed can be used by companies or laboratories that serve the mussel industry and authorities in order to respond to the traceability demands, particularly, to certify-authenticate the species of the raw material used.

### STATE OF THE ART

Salt water mussels (Mytilidae) are bivalve molluscs belonging to a family of great economical and gastronomical interest, including species of commercial importance belonging to the genus *Mytilus.* As other bivalves, these are filtering animals that live fixed to the substrate. They are exclusively marine animals and live both in intertidal zones and in submerged areas of the coastline from all over the world. Currently, so as to distinguish among the species within the genus *Mytilus,* the PCR-RFLP *Aci*l assay of the polyphenolic adhesive protein. This assay detects polymorphisms associated to each species, which allow identification of each of them. By polymorphism is understood any difference in the nucleotide sequence in the genome, wherein these differences may be: changes in the type of base, deletions (loss of one or more bases), insertions (addition of one or more bases) or duplicates.

The PCR-RFLP assay (RFLP- Restriction fragment length polymorphisms) consists of amplifying, by means of polymerase chain reaction (PCR), a specific segment of the genome of the individual so as to obtain multiple copies or amplicons of the initial segment. In order to detect polymorphisms (differences) in this amplicon, digestion is performed with suitable restriction enzymes and the size of the obtained fragments is determined by means of electrophoresis.

The adhesive protein gene codifying the protein allows adhesion of the mytilidae to the substrate and comprises a repetitive and non-repetitive region. By means of PCR-RFLP Acil of the polyphenolic adhesive protein, amplifying a segment of the non-repetitive region of this gene, polymorphisms associated to each species can be detected so as to differentiate the species *M. edulis* (180 pb amplicon) of *M. chilensis* and *M. galloprovincialis* (126 pb amplicon). In order to distinguish between these last two, the amplicons are digested with the Acil restriction enzyme, which only cuts the fragment of *M. galloprovincialis* in 75 and 51 pb fragments. The size of the different fragments will be visualized in agarose gels or polyacrylamide. The PCR-RFLP method, although being the mostly used, it is very laborious, slow and costly, since it includes enzyme digestion and two visualization steps of the amplicons in gels (agarose and polyacrylamide).

The application AU2013260747 of the prior art discloses a portable equipment for genetic identification including Polymerase Chain Reaction (PCR) and the High Resolution Melting (HRM) technique, which is configured for determining and communicating analysis and identification results. That is, it refers to portable equipment and not to a method for identification of species of the genus *Mytilus.* Likewise, it does not describe any primer, as it is described in the present invention.

Inque et al. teaches a method for detecting and identifying mussel species of the genus Mytilus comprising amplifying a segment of the gene encoding the polyphenolic adhesive protein Glu-5 by PCR using the primers Me15 and Me16 (KOJI INQUE et al. "Interspecific variations in adhesive protein sequences of Mytilus edulis, M. galloprovincialis and M. trossulus". BIOLOGICAL BULLETIN, LANCASTER PRESS, INC, LANCASTER PA, US, Vol 189, 3, 370-375, 1995). Besides, Larrain teaches a method for detecting and identifying mussel species of the genus Mytilus too, comprising amplifying a segment of the gene encoding the polyphenolic adhesive protein by PCR using the primers Me15 and Me16 and analysing the amplified fragments by Restriction Fragment Length Polymorphism RFLP (MARIA ANGÉLlCA LARRAíN et al. "Genetic composition of Mytilus species in mussel populations from southern Chile". LATIN AMERICAN JOURNAL OF AQUATIC RESEARCH, Vol 40, 1, 1077-1084, 2012). The primer pair used by either of these studies is however not very specific, and no suggestion is given in any of them about the analysis of the Glu-5 gene by PCR-HRM to identify mussel species.

Specifically, the present invention refers to specific primers as defined in claim 1, and methods as defined in claims 2-6 using said primers, that together with the PCR and HRM techniques allow identification of different species of economic importance within the genus *Mytilus.* No other document from the prior art describes the primers specified herein, nor discloses the steps of the method used in the present invention.

As it has been mentioned before, in order to identify the different species of mytilidae currently being of economic importance (*M. edulis, M. chilensis* and *M. galloprovincialis*)*,* the so called PCR-RFLP Acil assay of the adhesive polyphenolic protein is used, which takes at least two days and has a cost four times higher than the method disclosed in the present invention: HRM cost per sample: $821 (equivalent to USD 1.3); PCR-RFLP cost per sample: $3391 (equivalent to USD 5.5). The above is partly due to the value of one of the main reactants (the Acil restriction enzyme) and to the need of visualizing the results of two steps (PCR and RFLP) in gels. The greater cost and work difficult the massive application thereof in the authentication of species of the genus *Mytilus* and none of the documents of the prior art provides a solution to this problem.

In order to solve this problem of the conventional art used for identifying the species of the genus *Mytilus* (PCR-RFLP Acil of the polyphenolic adhesive protein), the present invention provides a method as defined in the appended claims for the identification of species from the genus *Mytilus* faster and cheaper.

Primers have been designed as well as a method using said primers in the polymerase chain reaction (PCR). The above, together with the HRM technique allows identifying a variation in the gene sequence of the polyphenolic adhesive protein which is diagnosed for mussel species *Mytilus chilensis, Mytilus galloprovincialis* and *Mytilus edulis.* All this allows fast identification of the species (in 20% of the time employed in the traditional assay), simple and at a cost which is four times cheaper than the traditional technology, generating different melting or dissociation curves, specific for each one of the species to be identified and easy to interpret; and without the need of visualizing the results in gels.

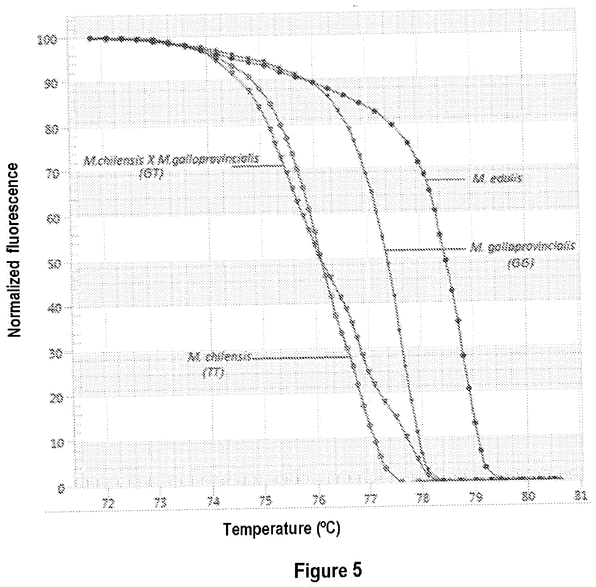

The present invention uses a technology based on the High Resolution Melting (HRM), this technique have been developed for detecting SNP - Single Nucleotide Polymorphism - in small amplicons, the main advantage thereof being that it is low cost and that it can be made in any laboratory having a thermocycler for real time PCR. Identification of the SNPs is achieved by using primers especially designed for amplifying the region of interest of the genome and intercalating fluorophores which can be saturation bonded to DNA without inhibiting PCR reaction, followed by an analysis of the dissociation ("melting") curve of the amplicons. Detection and identification of an SNP is produced because the generated dissociation curves are different for each SNP allele, since AT and GC pairs require different heat energy to break hydrogen bonds. The dissociation curves also detect polymorphisms having a size which is between amplicons (Figure 5).

### DESCRIPTION OF THE FIGURES

Figure 1. Sequence alignment of the polyphenolic adhesive protein gene of *M. galloprovincialis* and *M. chilensis.* Inside a rectangle: SNP found between *M. galloprovincialis* and *M. chilensis.*
Figure 2. Identification of the restriction enzyme Acil cleavage site.
Figure 3. Binding site of the primers PAPM and Me-15 to the polyphenolic adhesive protein of *M. galloprovincialis.*
Figure 4. Alignment for *M. edulis* with *M. chilensis, M. galloprovincialis* and PAPM primers.
Figura 5. Normalized HRM curve using PAPM primers. ▼ corresponds to *M. galloprovincialis* individuals (Genotype G/G, n=1), ∇ corresponds to *M. chilensis* X *M. galloprovincialis* hybrids (heterozygotes G/T, n=2), ○ corresponds to *M. chilensis* individuals (Genotype T/T, n=3) and • corresponds to *M. edulis* individuals (n=3). All the samples are in duplicate, except for the hybrids which are in triplicate.

### DESCRIPTION OF THE INVENTION

The present invention describes a set of primers designed for detection and identification of species from the genus Mytilus, such as for example, *Mytilus galloprovincialis, Mytilus chilensis,* and *Mytilus edulis.* This set of primers comprises a primer of SEQ ID No. 1, referred to as PAPM-SNP F and a primer of SEQ ID No. 2, referred to as PAPM-SNP R, referred to as PAPM out of the polyphenolic adhesive protein of the genus *Mytilus;*
PAPM-SNP F: 5'-GGAACAAAGCATGGACCA-3';
PAPM-SNP R: 5'-GACAGCTTCTTTGCAAGTGG-3'.

These primers recognize a gene section of the adhesive polyphenolic protein of said species, which is preserved therein, but which allows identifying one with respect to the other by means of PCR techniques, and analysis of dissociation curves (HRM).

The present invention also describes a method for detecting and identifying mussels species within the *Mytilus* genus in a fast and cheaper way, comprising the steps of:
a) mixing between 1-50 ng/µl of a DNA sample of the species to be identified with the following reactants required for PCR and HRM reaction; reagents from a fluorescence kit suitable for carrying out an analysis; between 0.05-0.5 µl of primers of SEQ ID No. 1 and between 0.05-0.5 µl of primers of SEQ ID No. 2 for a final concentration from 0.1-0.5 µM of each of them; a reference fluorophore, where necessary according to the equipment, and from 2-40 µL of PCR-grade H₂O (until completing the suitable volume according to the PCR equipment used);
b) amplifying a gene segment codifying the polyphenolic adhesive protein which is in the DNA of the mixture obtained in step a), with the primers of SEQ ID No. 1 and SEQ ID No. 2 by PCR under the following conditions: activation of Taq DNA polymerase at a temperature between 70-100°C for 1 to 10 minutes, 20 to 45 amplification cycles with a denaturation step at a temperature between 70-100°C for 10 to 60 seconds, primers binding at a temperature between 40-80°C for 10 seconds to one minute, and an extension step at the end of each cycle at a temperature between 50-80°C for 10 seconds to one minute and a final extension step at a temperature between 50-80°C for 1 to 10 minutes;
c) ending PCR with an extra cycle for constructing the dissociation curve with a temperature increase from 50° to 95°C between 1 to 15 minutes.
d) Generating, during step c), dissociation curves of the segments being amplified in step b) by means of HRM technique; and
e) Identifying the species which the sample belongs to from the dissociation curves obtained with the HRM technique, contrasting the results with control subjects of a known genotype.

### APPLICATION EXAMPLES

### EXAMPLE 1: Sequence analysis and primer design.

In order to design the suitable primers for identifying the species, differences are firstly observed in the polyphenolic adhesive protein sequences of the three species to be analysed. The sequence lengths for each species are: *M. edulis* 180 base pairs, *M. chilensis* and *M*. *galloprovincialis* 126 base pairs each. Therefore, differences between *M. chilensis* and *M. galloprovincialis* sequences must be identified since both of them have the same length.

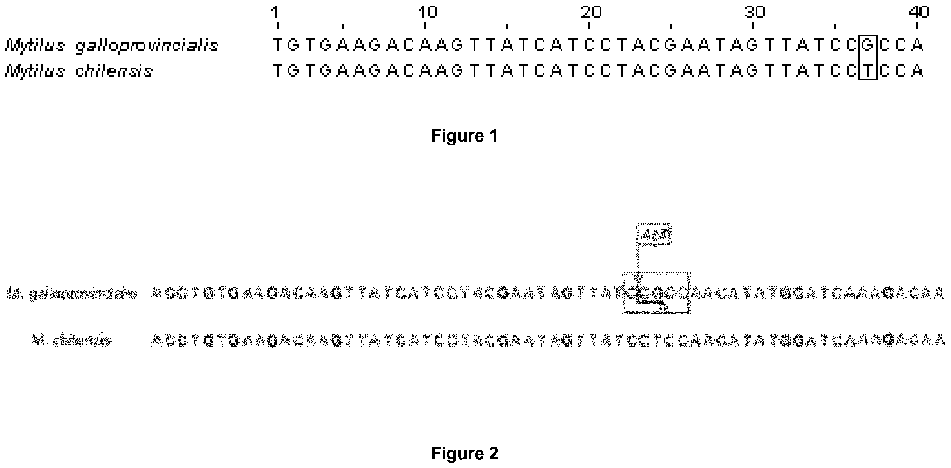

Thereby, sequences of the polyphenolic adhesive protein were compared from the two species of the genus *Mytilus; Mytilus galloprovincialis* (Genbank: D63778, SEQ ID No. 3) and *Mytilus chilensis* (Genbank: D0640609.1 SEQ ID No. 4), by sequence alignment (Figure 1).

In said Figure 1 it can be seen that there is a whole match in the sequences being compared, except for the presence of a SNP at position 66. In *M. galloprovincialis,* there is guanine, whereas in *M. chilensis* this nucleotide is changed by thymine (66G> T).

The presence of SNP 66G> T explains and allows identifying the Acil restriction enzyme cleavage site, that cuts the *M. galloprovincialis* amplicon into two fragments, whereas this does not happen in *M. chilensis.* Specifically, the Acil restriction enzyme recognizes cleavage sites of sequence 5'-CCGC-3', which can be observed in *M. galloprovincialis* and not in the *M*. *chilensis* sequence (Figure 2) where the change guanine by thymine, sequence 5'-CCGC-3', eliminates the Acil restriction enzyme cleavage site.

Then, new primers were designed being suitable for HRM technique referred to as PAPM-SNP F and PAPM-SNP R (SEQ ID No. 1 and SEO ID No. 2, respectively):
PAPM-SNP F: 5'-GGAACAAAGCATGGACCA-3';
PAPM-SNP R: 5'-GACAGCTTCTTTGCAAGTGG-3'.

By comparing these primers with those from PCR-RFLP method (referred to as Me-15 and Me-16), it is observed that the PAPM primers have a bigger melting or dissociation temperature ("tm") and better quality: they do not generate dimers and the amplificate is smaller, 116 bp vs 126 bp, which increases resolution of HRM technique (Table 1).

**Table 1: Comparison of the quality of primers from PCR-RFLP (Me15-16) vs primers designed in the present invention (PAPM).**

| **Parameter** | | | |
|---|---|---|---|
| **Primer** | **TM (°C)** | **% GC** | **Terminal auto dimer** |
| Me-15 | 51.9 | 40* | 0* |
| Me-16 | 50.1 | 29** | 117** |
| PAPM F | 55 | 50 | 0 |
| PAPM R | 54.3 | 50 | 0 |

| | | | |
|---|---|---|---|
| * Parameter categorized as bad **Parameter categorized as very bad | | | |

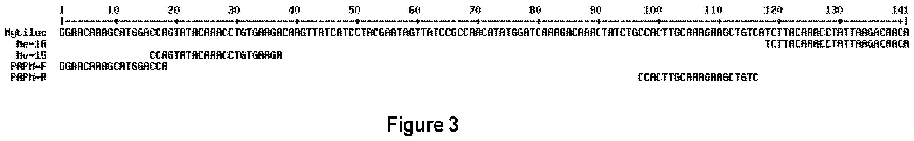

Figure 3 shows the binding site of the PAPM and Me15-16 primers in the *M. galloprovincialis* sequence. It is important to note that the binding site that each primer has is different and so, the amplificate obtained is also different using each one of them.

The *in-silico* amplificate of a gene segment of polyphenolic adhesive protein using PAPM primers, designed with the present application, corresponds to: SEQ ID No. 3 for *M*. *galloprovincialis,* SEQ ID No. 4 for *M. chilensis* and SEQ ID No. 5 for *M. edulis.*

In order to demonstrate specificity of the primers of the present invention, these were also analysed in other mussel species. Thus, a search was performed in the GenBank sequence database for polyphenolic adhesive protein sequences (Accession name; "polyphenolic adhesive protein"). This search result provided 53 results, from which 44 belong to some kind of mussel species; *M. galloprovincialis* (29), *M. chilensis* (10), *M. edulis* (3) and *M. trossulus* (2). The E value of the alignments was determined, indicating the probability that matches between both aligned sequences is by chance, and the identity percentage between them using BLAST tool, which is shown in Table 2. This result shows specificity of PAPM primers, which only amplify species of the genus *Mytilus,* which guarantees there will be no confusion with other species.

**Table 2: Specificity of PAPM primers in different species of the genus Mytilus.**

| Description | Bigger Score | Total score | % Coverage | E value | % Identity | Accession |
|---|---|---|---|---|---|---|
| *Mytilus sp.* JHX-2002 polyphenolic adhesive protein precursor | 209 | 209 | 100% | 6.00E-51 | 99% | AF489933.1 |
| *Mytilus galloprovincialis* mRNA for polyphenolic adhesive protein | 209 | 209 | 100% | 6.00E-51 | 99% | D63778.1 |
| *Mytilus galloprovincialis* Sample from Chile (Concepción) polyphenolic adhesive protein gene | 182 | 182 | 87% | 1.00E-42 | 99% | HQ257469.1 |
| *Mytilus galloprovincialis* Mgl1 polyphenolic adhesive protein gene | 182 | 182 | 87% | 1.00E-42 | 99% | DQ640590.1 |
| *Mytilus galloprovincialis* Sample from New Zealand (Akaroa) polyphenolic adhesive protein gene | 176 | 176 | 87% | 6.00E-41 | 98% | HQ257459.1 |
| *Mytilus chilensis* Mcl1 polyphenolic adhesive protein gene | 176 | 176 | 87% | 6.00E-41 | 98% | DQ640601.1 |
| *Mytilus galloprovincialis* Sample from Turkey (Izmir) polyphenolic adhesive protein gene | 171 | 171 | 84% | 3.00E-39 | 98% | HQ257470.1 |
| *Mytilus galloprovincialis* Sample from New Zealand (Kaikoura) polyphenolic adhesive protein gene | 171 | 171 | 87% | 3.00E-39 | 97% | HQ257468.1 |
| *Mytilus edulis* Mell2 polyphenolic adhesive protein gene | 121 | 121 | 63% | 3.00E-24 | 96% | DQ640586.1 |
| *Mytilus edulis* clon 21 adhesive protein 1 (fp-1) mRNA | 121 | 194 | 100% | 3.00E-24 | 96% | AY845258.1 |
| *Mytilus edulis* Mell4 polyphenolic adhesive protein gene | 117 | 117 | 63% | 3.00E-23 | 95% | DQ640587.1 |
| *Mvtilus edulis* polyphenolic adhesive protein qene | 115 | 194 | 100% | 1.00E-22 | 95% | X54422.1 |
| *Mytilus californianus* mRNA polyphenolic adhesive protein gene | 89.8 | 89.8 | 49% | 8.00E-15 | 95% | AY960602.1 |
| *Mytilus californianus* mRNA polyphenolic adhesive protein gene | 89.8 | 89.8 | 49% | 8.00E-15 | 95% | AY960601.1 |
| *Mytilus coruscus* mRNA polyphenolic adhesive protein gene | 73.1 | 73.1 | 49% | 8.00E-10 | 89% | 063777.1 |
| *Mytilus trossulus* mRNA polyphenolic adhesive protein gene | 71.3 | 71.3 | 35% | 3.00E-09 | 98% | 050553.1 |

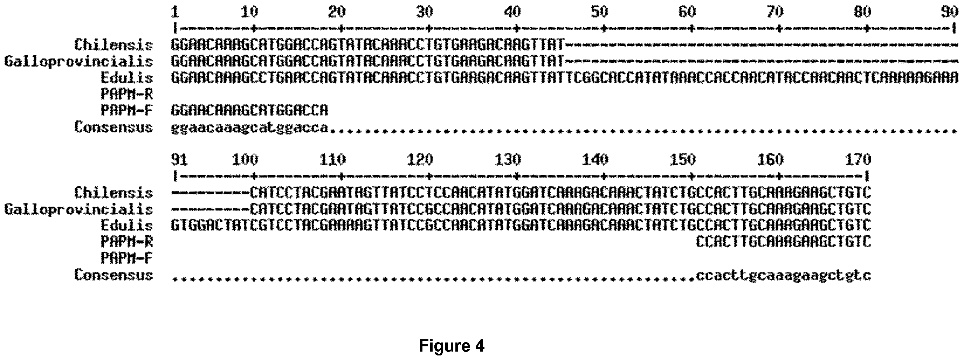

The *M. edulis* sequence was deeply analysed so as to determine if in this species there may be amplicon with the PAPM primers and if so, which expected length of the amplificate would be produced in the PCR reaction. Thus, the *M. edulis* sequence was aligned with the amplificate of PAPM primers in *M. chilensis* and *M. galloprovincialis,* together with the PAPM primer sequence (Figure 4). An almost perfect alignment was observed of the primers to *M. edulis* (except for a base in position 11 of PAPM F primer). The expected amplificate is 170 bp length, which matches the length observed when visualizing the PCR results in gels for this species, using the PAPM primers. A great insertion of 54 bp was observed prior to SNP 66 G> T (length polymorphism), detected using both the HRM technique (PAPM primers) and the PCR-RFLP method primers. The presence of this insertion is the reason why a bigger amplicon is generated, which allows identifying individuals from *M. edulis.*

### EXAMPLE 2: Implementation of the HRM technique for identifying species of the genus Mytilus.

With the purpose of obtaining comparative results, individuals were used that had been previously analysed with the method RFLP-PCR Acil currently in use (Inoue, Waite et al. 1995; Santaclara, Espineira et al. 2006). 425 mytilidae individuals of the above species were used, 308 were collected in the south of Chile, 46 being obtained from commercial samples from Galicia, Spain; 50 collected in Canada and 21 in Mexico (Table 3).

**Table 3. Origin of the Samples**

| **Country** | **Sampling location** | **n** | **Species according to PCR RFLP Me15-16 *Aci*l** |
|---|---|---|---|
| Chile | Isla Peel | 5 | *M. chilensis* |
| Chile | Metri | 54 | *M. chilensis* |
| Chile | Pichicolo | 54 | *M. chilensis* |
| Chile | La Arena | 53 | *M. chilensis* |
| Chile | Canutillar | 55 | *M. chilensis* |
| Chile | Canal Doldita | 54 | *M. chilensis* |
| Chile | Dichato | 3 | *M. chilensis x M. galloprovincialis* |
| Chile | Metri | 1 | *M. chilensis x M. galloprovincialis* |
| Chile | La Arena | 1 | *M. chilensis x M. galloprovincialis* |
| Chile | Canal Coldita | 1 | *M. chilensis x M. galloprovincialis* |
| Chile | Dichato | 27 | *M. galloprovincialis* |
| Spain | Galicia | 46 | *M. galloprovincialis* |
| Mexico | Rincón de las ballenas | 21 | *M. galloprovincialis* |
| Canada | Prince Edward Islands | 50 | *M. edulis* |

Both the PCR protocol, arrangement of samples on a plate, dissociation analysis and subsequent results analysis, were designed and realized using the program ECO™ Real-Time PCR System 4.0 (Illumina). The mixture Fast EvaGreen® qPCR Master Mix (Biotium) was used, said mixture containing EvaGreen®, Cheetah™ Taq DNA polymerase, dNTPs and a buffer, required for the PCR reaction, for reading and creating the dissociation curve. The tests were carried out using the following protocol (Table 4):

**Table 4; PCR mixture**

| **PCR-HRM** | **X1 (µl)** | **X48 (µl)** |
|---|---|---|
| 2x Fast Evagreen® | 4 | 192 |
| Primer of SEQ ID No. 1 | 0.075 | 3.6 |
| Primer of SEQ ID No. 2 | 0.075 | 3.6 |
| ROX | 0.1 | 4.8 |
| PCR-grade H₂O | 3.25 | 156 |
| Total | 7.5 0.5 | - |
| DNA | | - |
| Total + DNA | 8 | - |

The temperature profile programmed for the development of the PCR reaction with HRM was as follows: Cheetah™ Taq DNA polymerase activation for 2 minutes at 96°C, 45 amplification cycles with 15 seconds denaturation at 96°C and primer binding for a minute at 60°C, finishing with an extra cycle for construction of the dissociation curve (denaturation at 95°C for 15 seconds, hybridization for 15 seconds at 55°C and denaturation at 95°C for 15 seconds). A prior conventional PCR test, with the above mentioned protocol, was realized so as to discard the presence of some unspecific amplificate.

The HRM technique, as well as having the capacity to detect the different alleles of a SNP, allows detecting polymorphisms associated to length. As mentioned, using the *M. edulis* PAPM primers, amplicons will be generated having a length of 170 bp. This amplicon, since it is much bigger than that of *M. galloprovincialis* and *M. chilensis* (116 bp), has a dissociation curve being shifted towards a hotter area, this allowing identification of M. edulis individuals using the PAPM (Figure 5).

### EXAMPLE 3: Sequencing.

In order to verify that the SNP of interest would be within the amplificate being obtained using the PAPM primers, this was sequenced. First, a normal PCR reaction was performed with the PAPM primers, and then the amplicons were visualized in agarose gel so as to discard the presence of unspecific amplificates. Subsequently, the amplificates were purified using FavorPrep™ Gel/PCR purification MINI kit. The fragments thus obtained were sequenced by Macrogen Inc. The sequencing results indicated that the SNP of interest is within the amplicon generated with the PAPM primers.

## Claims

1. Set of primers for detecting and identifying mussel species of the genus *Mytilus,* **CHARACTERIZED in that** it comprises a primer of SEQ ID No. 1 and a primer of SEQ ID No. 2.

2. Method for detecting and identifying mussel species of the genus *Mytilus,* **CHARACTERIZED in that** it comprises the steps of:
a) mixing between 1-50 ng/µl of a DNA sample of the species to be identified with reactants for PCR and HRM reaction;
b) amplifying a gene segment codifying the polyphenolic adhesive protein which is in the DNA of the mixture obtained in step a), with the set of primers of claim 1;
c) ending PCR with an extra cycle for constructing the dissociation curve with a temperature increase from 50° to 95°C between 1 to 15 minutes;
d) Generating, during step c), dissociation curves of the sequence of the segments being amplified in step b) by means of HRM technique; and
e) Identifying the species which the sample belongs to from the dissociation curves obtained with the HRM technique, contrasting the results with control subjects of a known genotype.

3. Method according to claim 2, **CHARACTERIZED in that** the reactants mixed in step a) are the following: reagents from a fluorescence kit suitable for carrying out an HRM analysis; primers of SEQ ID No. 1 and SEQ ID No. 2; a reference fluorophore, if required, according to the type of equipment and completing the volume by addition of PCR-grade H₂O.

4. Method according to claim 3, **CHARACTERIZED in that** it is used between 0.05-0.5 µl of the primers of SEQ ID No. 1 and between 0.05-0.5 µl of the primers of SEQ ID No. 2 at a final concentration between 0.1-0.5 µM each.

5. Method according to claim 3, **CHARACTERIZED in that** it is used between 2-40 µl of PCR-grade H₂O until completing the appropriate volume according to the PCR equipment used.

6. Method according to claim 2, **CHARACTERIZED in that** amplification of step b) is carried out under the following conditions: activation of Taq DNA polymerase at a temperature between 70-100°C for 1 to 10 minutes, 20 to 45 amplification cycles with a denaturation step at a temperature between 70-100°C for 10 to 60 seconds, primers binding at a temperature between 40-80°C for 10 seconds to one minute, and an extension step at the end of each cycle at a temperature between 50-80°C for 10 seconds to one minute and a final extension step at a temperature between 50-80°C for 1 to 10 minutes.

## Patentansprüche

1. Primerset zur Erkennung und Identifizierung von Muschelarten der Gattung *Mytilus,* **dadurch GEKENNZEICHNET, dass** er einen Primer der SEQ ID NO: 1 und einen Primer der SEQ ID NO: 2 umfasst.

2. Verfahren zur Erkennung und Identifizierung von Muschelarten der Gattung *Mytilus,* **dadurch GEKENNZEICHNET, dass** es folgende Schritte umfasst:
a) Mischen von 1-50 ng/µL einer DNA-Probe der zu identifizierenden Art mit Reagenzien für die PCR- und HRM-Reaktion;
b) Amplifizieren eines Gensegments, das das polyphenolische adhäsive Protein kodiert, das sich in der DNA des in Schritt a) erhaltenen Gemischs befindet, mit dem Primerset gemäß Anspruch 1;
c) Beenden der PCR mit einem zusätzlichen Zyklus zur Erstellung der Dissoziationskurve mit einer Temperaturerhöhung von 50° auf 95°C zwischen 1 bis 15 Minuten;
d) Erzeugen, während Schritt c), von Dissoziationskurven der Sequenz der Segmente, die in Schritt b) amplifiziert werden, mittels HRM-Technik; und
e) Identifizieren der Art, zu der die Probe gehört, anhand der mit der HRM-Technik erhaltenen Dissoziationskurven, wobei die Ergebnisse mit Kontrollindividuen eines bekannten Genotyps abgeglichen werden.

3. Verfahren gemäß Anspruch 2, **dadurch GEKENNZEICHNET, dass** die in Schritt a) gemischten Reagenzien die folgenden sind: Reagenzien aus einem Fluoreszenzkit, das für die Durchführung einer HRM-Analyse geeignet ist; Primer der SEQ ID NO: 1 und SEQ ID NO: 2; ein Bezugsfluorophor, falls erforderlich, je nach Art der Einrichtung, und Auffüllen des Volumens durch Zugabe von H₂O in PCR-Qualität.

4. Verfahren gemäß Anspruch 3, **dadurch GEKENNZEICHNET, dass** zwischen 0,05-0,5 µL der Primer der SEQ ID NO: 1 und zwischen 0,05-0,5 µL der Primer der SEQ ID NO: 2 in einer Endkonzentration zwischen jeweils 0,1-0,5 µM verwendet werden.

5. Verfahren gemäß Anspruch 3, **dadurch GEKENNZEICHNET, dass** zwischen 2-40 µL H₂O in PCR-Qualität bis zum Auffüllen des geeigneten Volumens je nach der verwendeten PCR-Einrichtung verwendet wird.

6. Verfahren gemäß Anspruch 2, **dadurch GEKENNZEICHNET, dass** die Amplifikation von Schritt b) unter den folgenden Bedingungen durchgeführt wird: Aktivierung der Taq-DNA-Polymerase bei einer Temperatur zwischen 70-100°C für 1 bis 10 Minuten, 20 bis 45 Amplifikationszyklen mit einem Denaturierungsschritt bei einer Temperatur zwischen 70-100°C für 10 bis 60 Sekunden, Bindung der Primer bei einer Temperatur zwischen 40-80°C für 10 Sekunden bis eine Minute, und einem Elongationsschritt am Ende jedes Zyklus bei einer Temperatur zwischen 50-80°C für 10 Sekunden bis eine Minute und einem abschließenden Elongationsschritt bei einer Temperatur zwischen 50-80°C für 1 bis 10 Minuten.

## Revendications

1. Ensemble d'amorces pour la détection et l'identification des espèces de moules du genre *Mytilus,* **CARACTÉRISÉ en ce qu**'il comprend une amorce de la SEQ ID n° 1 et une amorce de la SEQ ID n° 2.

2. Méthode de détection et d'identification des espèces de moules du genre *Mytilus,* **CARACTÉRISÉE en ce qu**'elle comprend les étapes suivantes :
a) mélange de 1-50 ng/µl d'un échantillon d'ADN de l'espèce à identifier avec des réactifs pour la réaction PCR et HRM ;
b) l'amplification d'un segment de gène codant pour la protéine adhésive polyphénolique qui se trouve dans l'ADN du mélange obtenu à l'étape a), avec le jeu d'amorces de la revendication 1 ;
c) terminer la PCR par un cycle supplémentaire pour construire la courbe de dissociation avec une augmentation de la température de 50° à 95°C entre 1 et 15 minutes ;
d) Générer, au cours de l'étape c), des courbes de dissociation de la séquence des segments amplifiés à l'étape b) au moyen de la technique HRM ; et
e) Identification de l'espèce à laquelle appartient l'échantillon à partir des courbes de dissociation obtenues avec la technique de HRM, en comparant les résultats avec des sujets témoins d'un génotype connu.

3. Méthode selon la revendication 2, **CARACTÉRISÉ en ce que** les réactifs mélangés à l'étape a) sont les suivants : réactifs provenant d'un kit de fluorescence approprié pour effectuer une analyse par HRM ; amorces de SEQ ID n° 1 et SEQ ID n° 2 ; un fluorophore de référence, si nécessaire, selon le type d'équipement et complétant le volume par l'ajout de H₂O de qualité PCR.

4. Méthode selon la revendication 3, **CARACTÉRISÉ en ce qu**'elle est utilisée entre 0,05-0,5 µl des amorces de la SEQ ID n° 1 et entre 0,05-0,5 µl des amorces de la SEQ ID n° 2 à une concentration finale comprise entre 0,1-0,5 µM chacune.

5. Méthode selon la revendication 3, **CARACTÉRISÉ en ce qu**'elle est utilisée entre 2 et 40 µl de H₂O de qualité PCR jusqu'à ce que le volume approprié soit atteint selon l'équipement PCR utilisé.

6. Méthode selon la revendication 2, **CARACTÉRISÉ en ce que** l'amplification de l'étape b) est effectuée dans les conditions suivantes : activation de l'ADN polymérase Taq à une température comprise entre 70 et 100°C pendant 1 à 10 minutes, 20 à 45 cycles d'amplification avec une étape de dénaturation à une température comprise entre 70 et 100°C pendant 10 à 60 secondes, des amorces se liant à une température comprise entre 40 et 80°C pendant 10 secondes à une minute, et une étape d'extension à la fin de chaque cycle à une température comprise entre 50 et 80°C pendant 10 secondes à une minute et une étape finale d'extension à une température comprise entre 50 et 80°C pendant 1 à 10 minutes.
